# EUROPEAN PATENT SPECIFICATION

(11) **EP 2 122 180 B1**
(45) Date of publication and mention of the grant of the patent: **14.06.2017**
(21) Application number: 07859009.8
(22) Date of filing: 13.12.2007
(51) Int. Cl.: F04D 25/06, F04D 29/10, F04D 29/58, H02K 9/00, F04D 25/08, A47L 5/22

(54) **FAN FOR AN APPARATUS FOR THE REGULATED DELIVERY OF A GAS, IN PARTICULAR OXYGEN**
GEBLÄSE FÜR EINE VORRICHTUNG ZUR REGULIERTEN ZUFUHR EINES GASES, INSBESONDERE SAUERSTOFF
VENTILATEUR POUR UN APPAREIL POUR DISTRIBUTION RÉGULÉE D'UN GAZ, EN PARTICULIER D'OXYGÈNE

(30) Priority: 18.12.2006 FR 0610992; 18.12.2006 US 875505 P; 07.02.2007 US 899997 P; 13.02.2007 FR 0701001
(43) Date of publication of application: 25.11.2009
(73) Proprietor: Airfan, 31770 Colomiers (FR)
(72) Inventor: GRASMUCK, Gilbert, 31880 La Salvetat Saint Gilles (FR)
(74) Representative: Junca, Eric
(86) International application number: PCT/IB2007/003874
(87) International publication number: WO 2008/075158

(56) References cited:
- WO-A-2006/131470
- FR-A1- 2 391 691
- US-A- 4 527 960

## Description

The present invention relates to a fan used in an apparatus for the regulated delivery of a gas, in particular oxygen. This apparatus may especially be a respiratory assistance apparatus.

WO 2006/131470 is considered as the closest prior art to the subject-matter of claim 1.

A fan delivering gases other than air, especially oxygen, conventionally comprises a volute whose inlet opening is in communication with the oxygen feed line, an impeller situated immediately downstream of this inlet opening and comprising an inlet opening connected to this inlet opening of the volute and outlet orifices emerging into the volute, and an electric motor for rotating the impeller.

The rotation of the impeller generates a centrifugal oxygen flow which is converted into increased gas pressure in the volute.

There is a major problem in this type of fan of oxygen leaking in the direction of the electric motor through the gap which necessarily exists between the shaft of the motor and the wall of the volute that is traversed by this shaft. Oxygen penetration in the direction of the motor or other electric elements or components of the apparatus resulting from such a leak is not acceptable on account of the risk of ignition which could result therefrom; moreover, the standards of a certain number of countries prohibit the presence of oxygen in a proportion of more than 26% in those regions of a medical apparatus that include electric circuits.

The main objective of the invention is to solve this problem under the best conditions.

There is also a problem of cooling the electric motor in the existing fans, this motor being subject to significant heating on account of its high speed of rotation.

Another objective of the invention is to overcome this problem under the best conditions, in combination with tackling the aforementioned leakage problem.

The fan in question comprises, in a manner known per se, a volute whose inlet opening is in communication with the oxygen feed line, an impeller situated immediately downstream of the inlet opening of the volute and comprising an inlet opening connected to this inlet opening of the volute and outlet orifices emerging into the volute, and an electric motor for rotating the impeller.

According to the invention, the fan comprises:
- a compartment in which the motor and, where appropriate, other electric elements or components belonging to the fan are placed, this compartment comprising an end wall traversed by the shaft of the motor and being supplied with pressurized air, and
- a leak collection chamber situated intermediately between the volute and the said compartment, this chamber likewise being traversed by the shaft of the motor and including a discharge orifice.

Thus, the motor and any other electric elements or components of the fan are placed in a compartment containing pressurized air, this air flowing out of this compartment through the gap which necessarily exists between the shaft of the motor and the said end wall. This flow of pressurized air makes it possible to oppose the ingress into the compartment of pressurized oxygen flowing through the gap which necessarily exists between the shaft of the motor and the wall of the volute that is traversed by this shaft.

These respective air and oxygen leaks are collected in the collection chamber and can be discharged by simply flowing through a discharge orifice belonging to this collection chamber.

Preferably, the shaft of the motor comprises at least one deflector member for separating the respective air and oxygen flows and for orienting these flows, on leaving the said gaps, in directions which are substantially perpendicular to the shaft of the motor.

Disturbances between these flows are thus eliminated, and the risk of oxygen penetrating into the compartment is also reduced, notwithstanding the pressure of the oxygen flow, which is substantially higher than that of the air flow.

The air flowing into the compartment can be advantageously pressurized by an impeller arranged opposite the air inlet opening belonging to this compartment. Advantageously, in this case, this impeller is arranged on the drive shaft for the impeller situated in the volute. It is therefore driven by the same motor.

The walls defining the compartment can comprise one or more openings emerging directly into the said collection chamber.

This opening or these openings allow a flow of pressurized air around the motor and then into the collection chamber, thereby making it possible to cool the motor. The flow of this air into the collection chamber additionally allows a rapid discharge of the mixture of air and oxygen present in this chamber.

Preferably, the compartment comprises an internal transverse wall which is distinct from the aforementioned end wall and which is perforated by at least one hole for the flow of air across this wall.

This internal transverse wall and this end wall thus define between them a secondary air flow chamber, and the hole or holes belonging to the said internal transverse wall optimize the pressurization of this secondary chamber so as to allow the leak at the said gap to occur under the best conditions. This leakage rate thus remains effective, using the relatively high air pressure created in the secondary chamber.

The said internal transverse wall can in particular be formed integrally with other walls defining the compartment and serve to centre the motor in this compartment.

The said end wall can be composed of a thin plate.

With the same aim, the wall of the volute that is traversed by the shaft of the motor can also be composed of a thin plate adapted to the precision required for obtaining a small clearance at the level of the said gap situated where the shaft passes into the volute wall.

The leaks collected by the collection chamber can be purely and simply discharged, or can be recycled, in which case the discharge orifice of the collection chamber can be connected directly to the oxygen feed line.

In this same case of recycling the collected leaks, the collection chamber is advantageously divided into two adjoining subchambers, one of which, situated adjacent to the volute, is mainly intended to collect the flow of oxygen coming from this volute and the other of which, situated adjacent to the said compartment, is mainly intended to collect the flow of air coming from this compartment, each subchamber comprising a discharge orifice which is specific to it.

Thus, the subchamber situated adjacent to the volute mainly collects the oxygen flow, which can be recycled by placing the discharge orifice belonging to this subchamber in communication with the oxygen feed line.

The invention will be properly understood and other features and advantages thereof will become apparent with reference to the appended schematic drawing which represents, by way of non-limiting examples, a number of possible embodiments of the apparatus to which it relates.
Figure 1 is a view in axial section thereof, according to a first embodiment;
Figure 2 is a detail view thereof, on an enlarged scale;
Figure 3 is a view in axial section thereof, similar to Figure 1, according to a second embodiment;
Figure 4 is a detail view thereof, on an enlarged scale, according to this second embodiment;
Figure 5 is a view in axial section thereof, similar to Figure 1, according to a third embodiment; and
Figure 6 is a detail view thereof, on an enlarged scale, according to this third embodiment.

For reasons of simplification, those parts or elements of one embodiment which reappear in an identical or similar manner in another embodiment are designated by the same reference numbers and are not described again.

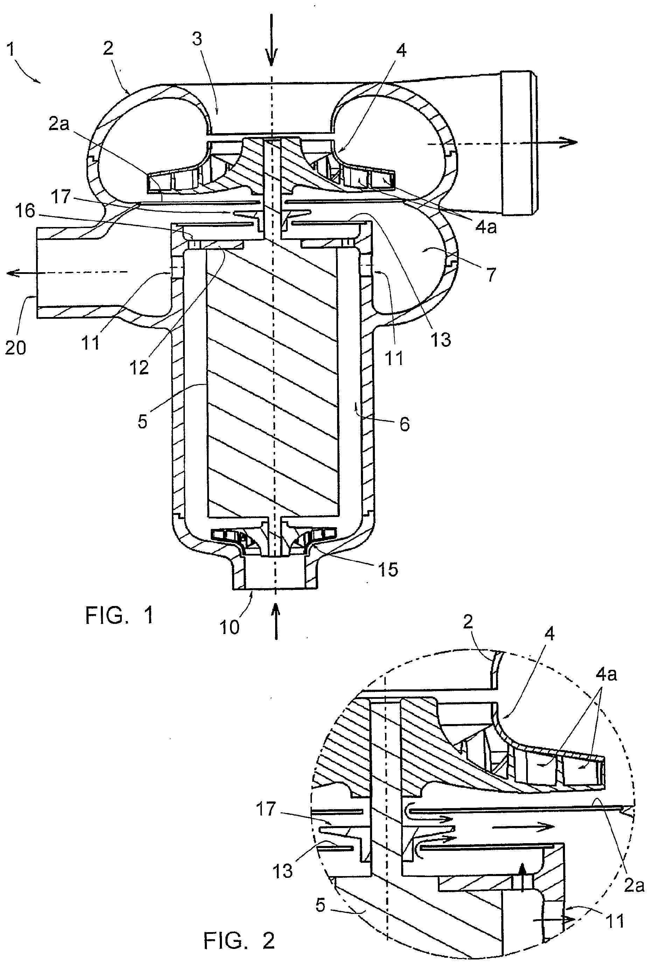

Figures 1 and 2 represent a fan 1 used in an apparatus for the regulated delivery of a gas, in particular oxygen, it being possible especially for this apparatus to be a respiratory assistance apparatus.

The fan 1 conventionally comprises a volute 2 whose inlet opening 3 is in communication with an oxygen feed line (not shown in this figure), an impeller 4 situated immediately downstream of this inlet opening 3 and comprising an inlet opening connected to this opening 3 and outlet orifices 4a emerging into the volute 2, and an electric motor 5 for rotating the impeller 4.

The bottom of the volute 2 is defined by a wall composed of a thin plate 2a traversed by the shaft of the motor 5. There is a gap between this shaft and this plate 2a, through which pressurized oxygen contained in the volute 2 inevitably flows.

As is represented, the fan 1 also comprises a compartment 6 and a leak collection chamber 7.

The compartment 6 contains the motor 5 and also, where appropriate, other electric elements or components belonging to the fan 1. It comprises an air inlet 10, a number of openings 11 emerging directly into the collection chamber 7, a transverse wall 12 and an end wall 13.

The air inlet 10 is situated on that end of the compartment 6 which is opposed to the volute 2, and is coaxial with the shaft of the motor 5 including the impeller 4. Immediately downstream of this inlet 10, the fan 1 comprises an impeller 15, smaller than the impeller 4, arranged on an extension of the shaft of the motor 5 which includes the impeller 4.

As will be understood, the rotation of this impeller 15 by the motor 5 makes it possible to create a pressurized air circulation in the compartment 6; this air passes into the collection chamber 7, for the most part through openings 11, and for the minor part through holes 16 belonging to the wall 12 and through the gap which exists between the hub of a deflector member 17 belonging to the shaft of the motor 5 and the end wall 13.

In this illustrated example, the openings 11 are formed in the peripheral wall defining the compartment 6, perpendicularly to the shaft of the motor 5.

The transverse wall 12 is formed integrally with this peripheral wall and serves to centre the motor 5 in the compartment 6.

The end wall 13 for its part is composed of a thin plate. This wall 13 is fastened to a rim belonging to the fan 1 in the continuation of the peripheral wall defining the compartment 6, such that it is located at a distance from the wall 12 and such that it defines a secondary air flow chamber therewith. The holes 16 belonging to the transverse wall 12 optimize the pressurization of this secondary chamber so as to allow an air leak at the aforementioned gap.

The collection chamber 7 is situated between the volute 2 and the compartment 6, encompassing the upper part thereof. It is traversed by the shaft of the motor 5 and includes a discharge orifice 20.

The deflector member 17 comprises, in addition to the aforementioned hub which serves to mount it on the shaft of the motor 5, a part in the form of a flange formed integrally with this hub. As is shown more particularly in Figure 2, this deflector member 17 makes it possible to separate the respective air and oxygen flows coming from the aforementioned respective gaps and to orient these flows, on leaving the said gaps, in directions which are substantially perpendicular to the shaft of the motor 5.

As is apparent from the foregoing, the air flowing out of the compartment 6 through the gap which exists between the shaft of the motor 5 and the end wall 13 makes it possible to oppose the ingress into the compartment 6 of pressurized oxygen flowing through the gap which exists between the shaft of the motor 5 and the wall 2a of the volute 2 that is traversed by this shaft. These respective air and oxygen leaks are collected in the chamber 7 and can be discharged by simply flowing through the discharge orifice 20 belonging thereto.

The deflector member 17 makes it possible to eliminate the disturbances between these flows.

Moreover, the impeller 15 makes it possible to achieve a forced circulation of pressurized air around the motor 5, thereby providing good cooling for the latter. The flow of this air into the collection chamber 7 additionally allows a rapid discharge of the mixture of air and oxygen present in this chamber.

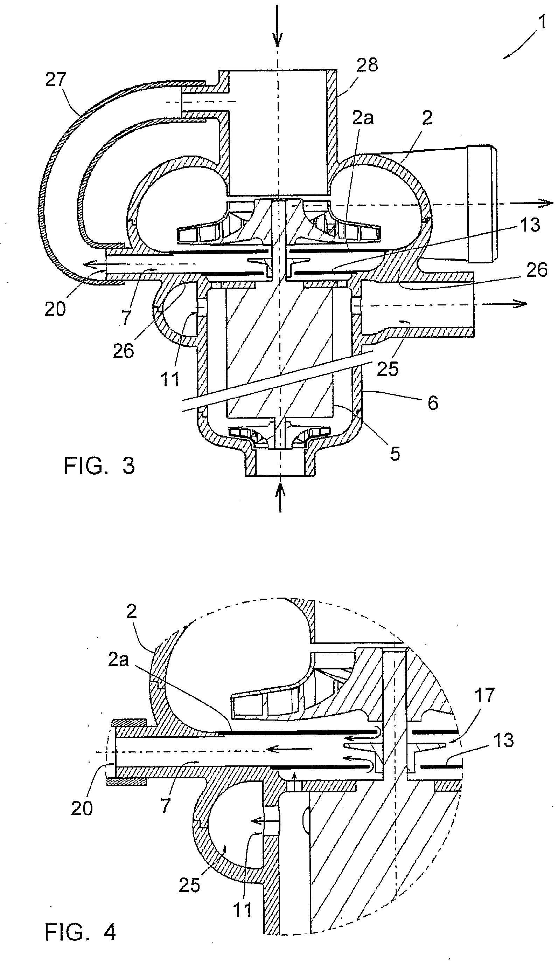

Figures 3 and 4 represent an apparatus 1 similar to the one which has just been described apart from the fact that, in this case, the orifices 11 emerge into a chamber 25 separated from the collection chamber 7. The latter is limited to the region included between the wall 2a defining the bottom of the volute 2, the end wall 13 and walls 26 formed by the body of the apparatus 1, joining that part of the compartment 6 situated adjacent to the volute 2 to the peripheral wall defining the chamber 7.

The discharge orifice 20 belonging to the collection chamber 7 is in communication, via a pipe 27, with the oxygen feed line 28. The mixture of air and oxygen flowing through the aforementioned gaps is thus recycled in the oxygen circuit.

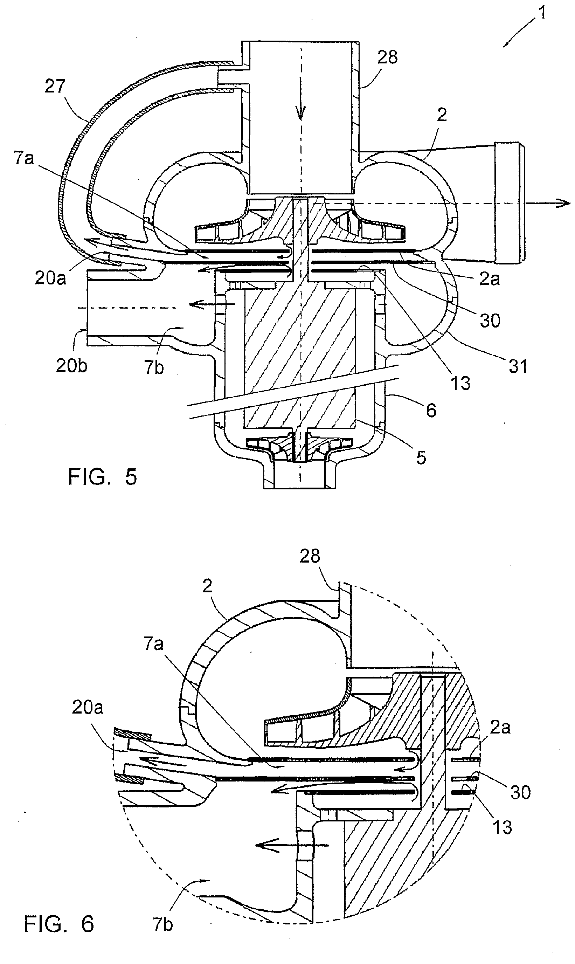

Figures 5 and 6 represent an apparatus 1 in which the collection chamber 7 is divided into two adjoining subchambers 7a, 7b, one, 7a, of which is defined by the wall 2a of the volute 2 and by an intermediate wall 30, and the other, 7b, of which is defined by the intermediate wall 30 and by a peripheral wall 31 attaching to the compartment 6.

As will be understood with reference to these Figures 5 and 6, the subchamber 7a is mainly intended for collecting the oxygen flow coming from the volute 2, whereas the subchamber 7b is mainly intended for collecting the air flow coming from the compartment 6. Each subchamber 7a, 7b comprises a discharge orifice 20a, 20b which is specific to it, that of the subchamber 7a being connected, by a pipe 27, to the oxygen feed line 28. Virtually pure oxygen is thus recycled in the oxygen circuit.

The invention therefore provides a fan 1 which makes it possible under the best conditions to solve the problem of oxygen leakage in the direction of the motor 5, oxygen penetration not being acceptable on account of the risk of ignition which could result therefrom, and to simultaneously solve the problem of cooling the motor, this motor being subject to significant heating on account of its high speed of rotation.

It goes without saying that the invention is not limited to the embodiments described above by way of examples but that it extends to all the embodiments covered by the claims appended hereto.

## Claims

1. Fan (1) used in an apparatus for the regulated delivery of a gas, in particular oxygen, comprising a volute (2) whose inlet opening (3) is in communication with an oxygen feed line, an impeller (4) situated immediately downstream of the inlet opening (3) of the volute (2) and comprising an inlet opening connected to this inlet opening (3) of the volute (2) and outlet orifices (4a) emerging into the volute (2), and an electric motor (5) for rotating the impeller (4), **characterized in that** it comprises:
- a compartment (6) in which the motor (5) and, where appropriate, other electric elements or components belonging to the fan (1) are placed, this compartment (6) comprising an end wall (13) traversed by the shaft of the motor (5) and being supplied with pressurized air, and
- a leak collection chamber (7) situated intermediately between the volute (2) and the said compartment (6), this chamber likewise being traversed by the shaft of the motor (5) and comprising a discharge orifice (20).

2. Fan (1) according to Claim 1, **characterized in that** the shaft of the motor (5) comprises at least one deflector member (17) for separating the respective air and oxygen flows and for orienting these flows in directions which are substantially perpendicular to the shaft of the motor (5).

3. Fan (1) according to Claim 1 or Claim 2, **characterized in that** it comprises an impeller (15) arranged opposite the air inlet opening (10) belonging to the compartment (6).

4. Fan (1) according to Claim 3, **characterized in that** the impeller (15) belonging to the compartment (6) is arranged on the drive shaft for the impeller (4) situated in the volute (2), and is driven by the same motor (5) as the latter.

5. Fan (1) according to one of Claims 1 to 4, **characterized in that** the walls defining the compartment (6) comprise one or more openings (11) emerging directly into the said collection chamber (7).

6. Fan (1) according to one of Claims 1 to 5, **characterized in that** the compartment (6) comprises an internal transverse wall (12) which is distinct from the said end wall (13) and which is perforated by at least one hole (16) for the flow of air across this wall.

7. Fan (1) according to Claim 6, **characterized in that** the said internal transverse wall (12) is formed integrally with other walls defining the compartment (6) and serves to centre the motor (5) in this compartment (6).

8. Fan (1) according to one of Claims 1 to 7, **characterized in that** the said end wall (13) is composed of a thin plate.

9. Fan (1) according to one of Claims 1 to 8, **characterized in that** the wall (2a) of the volute (2) that is traversed by the shaft of the motor (5) is composed of a thin plate.

10. Fan (1) according to one of Claims 1 to 9, **characterized in that** the collection chamber (7) is divided into two adjoining subchambers (7a, 7b), one of which, situated adjacent to the volute (2), is mainly intended to collect the flow of oxygen coming from this volute (2) and the other of which, situated adjacent to the said compartment (6), is mainly intended to collect the flow of air coming from this compartment (6), each subchamber (7a, 7b) comprising a discharge orifice (20a, 20b) which is specific to it.

## Patentansprüche

1. Gebläse (1), das in einer Vorrichtung zur geregelten Zufuhr eines Gases, insbesondere Sauerstoff, verwendet wird und ein Spiralgehäuse (2), dessen Einlassöffnung (3) in Kommunikation mit einer Sauerstoffspeiseleitung steht, ein Laufrad (4), das sich unmittelbar stromabwärts der Einlassöffnung (3) des Spiralgehäuses (2) befindet und eine mit dieser Einlassöffnung (3) des Spiralgehäuses (2) verbundene Einlassöffnung und in das Spiralgehäuse (2) austretende Auslassöffnungen (4a) umfasst, und einen Elektromotor (5) zum Drehen des Laufrades (4) umfasst, **dadurch gekennzeichnet, dass** es umfasst:
- einen Raum (6), in welchem der Motor (5) und gegebenenfalls weitere elektrische Elemente oder Komponenten, die zu dem Gebläse (1) gehören, angeordnet sind, wobei dieser Raum (6) eine Endwand (13) umfasst, die von der Welle des Motors (5) durchquert wird, und mit Druckluft versorgt wird, und
- eine Leckage-Sammelkammer (7) die sich in einer Zwischenposition zwischen dem Spiralgehäuse (2) und dem Raum (6) befindet, wobei diese Kammer gleichfalls von der Welle des Motors (5) durchquert wird und eine Austrittsöffnung (20) umfasst.

2. Gebläse (1) nach Anspruch 1, **dadurch gekennzeichnet, dass** die Welle des Motors (5) wenigstens ein Ablenkelement (17) zum Trennen des Luft- und des Sauerstoffstroms und zum Lenken dieser Ströme in Richtungen, welche im Wesentlichen senkrecht zur Welle des Motors (5) sind, umfasst.

3. Gebläse (1) nach Anspruch 1 oder Anspruch 2, **dadurch gekennzeichnet, dass** es ein Laufrad (15) umfasst, das gegenüber der Lufteinlassöffnung (10) angeordnet ist, die zu dem Raum (6) gehört.

4. Gebläse (1) nach Anspruch 3, **dadurch gekennzeichnet, dass** das zu dem Raum (6) gehörende Laufrad (15) auf der Antriebswelle für das im Spiralgehäuse (2) befindliche Laufrad (4) angeordnet ist und von demselben Motor (5) wie das Letztere angetrieben wird.

5. Gebläse (1) nach einem der Ansprüche 1 bis 4, **dadurch gekennzeichnet, dass** die den Raum (6) definierenden Wände eine oder mehrere Öffnungen (11) umfassen, die direkt in die Sammelkammer (7) austreten.

6. Gebläse (1) nach einem der Ansprüche 1 bis 5, **dadurch gekennzeichnet, dass** der Raum (6) eine innere Querwand (12) umfasst, welche von der Endwand (13) verschieden ist und welche von wenigstens einem Loch (16) für den Durchfluss von Luft durch diese Wand durchbrochen wird.

7. Gebläse (1) nach Anspruch 6, **dadurch gekennzeichnet, dass** die innere Querwand (12) einstückig mit anderen Wänden ausgebildet ist, die den Raum (6) definieren, und dazu dient, den Motor (5) in diesem Raum (6) zu zentrieren.

8. Gebläse (1) nach einem der Ansprüche 1 bis 7, **dadurch gekennzeichnet, dass** die Endwand (13) aus einer dünnen Platte besteht.

9. Gebläse (1) nach einem der Ansprüche 1 bis 8, **dadurch gekennzeichnet, dass** die Wand (2a) des Spiralgehäuses (2), welche von der Welle des Motors (5) durchquert wird, aus einer dünnen Platte besteht.

10. Gebläse (1) nach einem der Ansprüche 1 bis 9, **dadurch gekennzeichnet, dass** die Sammelkammer (7) in zwei aneinander angrenzende Teilkammern (7a, 7b) aufgeteilt ist, von denen eine, die dem Spiralgehäuse (2) benachbart angeordnet ist, hauptsächlich dazu bestimmt ist, den aus diesem Spiralgehäuse (2) kommenden Strom von Sauerstoff zu sammeln, und von denen die andere, die dem Raum (6) benachbart angeordnet ist, hauptsächlich dazu bestimmt ist, den aus diesem Raum (6) kommenden Strom von Luft zu sammeln, wobei jede Teilkammer (7a, 7b) eine Austrittsöffnung (20a, 20b) umfasst, welche für sie spezifisch ist.

## Revendications

1. Ventilateur (1) utilisé dans un appareil de délivrance régulée d'un gaz, notamment d'oxygène, comprenant une volute (2) dont l'ouverture d'entrée (3) est en communication avec le conduit d'amenée d'oxygène, une roue à ailettes (4), située immédiatement en aval de l'ouverture d'entrée (3) de la volute (2), comprenant une ouverture d'entrée reliée à cette ouverture d'entrée (3) de la volute (2) et des orifices de sortie (4a) débouchant dans la volute (2), et un moteur électrique (5) d'entraînement de la roue (4) en rotation, **caractérisé en ce qu'**il comprend :
- un compartiment (6) dans lequel est placé le moteur (5) et éventuellement d'autres organes ou composants électriques que comprend le ventilateur (1), ce compartiment (6) comprenant une paroi d'extrémité (13) traversée par l'arbre du moteur (5) et étant alimenté en air sous pression, et
- une chambre (7) de collecte de fuites, intermédiaire entre la volute (2) et ledit compartiment (6), également traversée par l'arbre du moteur (5), et comprenant un orifice d'évacuation (20).

2. Ventilateur (1) selon la revendication 1, **caractérisé en ce que** l'arbre du moteur (5) comprend au moins un organe déflecteur (17) permettant de séparer les écoulements respectifs d'air et d'oxygène et d'orienter ces écoulements selon des directions sensiblement perpendiculaires à l'arbre du moteur (5).

3. Ventilateur (1) selon la revendication 1 ou la revendication 2, **caractérisé en ce qu'**il comprend une roue à ailettes (15) disposée en regard de l'ouverture (10) d'entrée d'air que comprend le compartiment (6).

4. Ventilateur (1) selon la revendication 3, **caractérisé en ce que** la roue à ailettes (15) que comprend le compartiment (6) est disposée sur l'arbre d'entraînement de la roue à ailettes (4) située dans la volute (2), et est entraînée par le même moteur (5) que cette dernière.

5. Ventilateur (1) selon l'une des revendications 1 à 4, **caractérisé en ce que** les parois délimitant le compartiment (6) comprennent une ou plusieurs ouvertures (11) débouchant directement dans ladite chambre de collecte (7).

6. Ventilateur (1) selon l'une des revendications 1 à 5, **caractérisé en ce que** le compartiment (6) comprend une paroi transversale interne (12) distincte de ladite paroi d'extrémité (13), qui est percée d'au moins un trou (16) d'écoulement de l'air au travers d'elle.

7. Ventilateur (1) selon la revendication 6, **caractérisé en ce que** ladite paroi transversale interne (12) forme corps avec d'autres parois délimitant le compartiment (6) et sert au centrage du moteur (5) dans ce compartiment (6).

8. Ventilateur (1) selon l'une des revendications 1 à 7, **caractérisé en ce que** ladite paroi extrémité (13) est constituée par une plaquette mince.

9. Ventilateur (1) selon l'une des revendications 1 à 8, **caractérisé en ce que** la paroi (2a) de la volute (2) traversée par l'arbre du moteur (5) est constituée par une plaquette mince.

10. Ventilateur (1) selon l'une des revendications 1 à 9, **caractérisé en ce que** la chambre de collecte (7) est divisée en deux sous-chambres contiguës (7a, 7b), dont une, située du côté de la volute (2), est principalement destinée à collecter l'écoulement d'oxygène provenant de cette volute (2) et dont l'autre, située du côté dudit compartiment (6), est principalement destinée à collecter l'écoulement d'air provenant de ce compartiment (6), chaque sous-chambre (7a, 7b) comprenant un orifice d'évacuation (20a, 20b) qui lui est propre.
